# EUROPEAN PATENT APPLICATION

(11) **EP 2 261 316 A1**
(43) Date of publication of application: **15.12.2010**
(21) Application number: 09162628.3
(22) Date of filing: 12.06.2009
(51) Int. Cl.: C12M 1/26, G01N 1/22

(54) **Method and device for determining the microbiological contamination in an environment**

(71) Applicant: Nutricion Infantil Funcional y Segura A.I.E., 08028 Barcelona (ES)
(72) Inventor: Rivero Urgell, Montserrat, 08035, Barcelona (ES); Gonzales Rivas, Fabián, 08292, Esparreguera (Barcelona) (ES); Marin de Mateo, Mercedes, 08193, Sant Cugat del Vallès (Barcelona) (ES)
(74) Representative: ZBM Patents

(57) **Abstract**

The method comprises providing at least one sampling plate (100) and at least one supporting member (20) in said environment with the supporting member being suitable for detachably receiving the sampling plate (100); and removing the plate (100) from the supporting member (20) after a period of time such that the microbiological contamination in said environment can be determined by analyzing said sampling plate (100). A device is provided for carrying out said method comprising the supporting member carrying one or more sampling plates, attaching means for detachably fixing the sampling plates therein and releasing means (50, 51, 52) for removing of the sampling plate (100) from the supporting member (20).

## Description

### FIELD OF THE INVENTION

The present invention relates to a method for determining the microbiological contamination in an environment in which at least one supporting member is provided in the environment to be analyzed. Such an environment may be, for example, in foodstuff processing facilities, restaurants, industries, hospitals, and it is intended to include objects, surfaces, etc and, in general, any target item to be analyzed for microbiological contamination.

The determination of the microbiological contamination in such an environment according to the invention is useful, e.g., for controlling contamination, maintaining a hygienic state, etc.

The invention further refers to a device for determining the microbiological contamination in an environment with which said method can be carried out.

### BACKGROUND ART

The microbiological contamination in a given environment can be determined and monitored by different known techniques.

One known technique is through the use of swabs dipped in a suitable sterile liquid and rubbed over the test surface. The number of microorganisms that are present on the swab can be then determined by subculturing to media. This technique has the main disadvantage of a low recovery rate of bacteria.

A further example is the contact plate method. This is carried out by obtaining a replicate from a surface in the environment to be examined using surface contact plates such as special Petri dishes containing a sterile growth medium (Rodac Plates) as disclosed, for example, in US5695988, US5854065, or GB1379506. The microorganisms on the surface of the plate stick to the growth medium surface and grow upon incubation. Determination of the number of viable microorganisms on the surface is thus made possible. However, this method has the disadvantage of the undesirable high cost of the Rodac plates as well as not being suitable for heavily contaminated surfaces.

There exist other methods for assessing the microbiological contamination of surfaces such as the sticky film method consisting in pressing a sticky film or tape against the surface to be examined and pressing sticky the exposed side on an agar plate (however it has been shown as being less effective than other methods in recovering bacteria from certain surfaces); or the use of ultrasonic devices (although with this method the surfaces to be examined must be small in size and removable so that they can be placed inside a container immersed in diluents); as well as the spray method consisting in applying a spray of washing solution against a circumscribed area of surface and the subsequent plating of the washing solution (this having the disadvantage of requiring a source of air pressure).

Still a further method for assessing the microbiological contamination in a surface in a given environment is the use of small model surfaces, e.g. in the form of a convex shaped plate. In this respect, ES2204326 provides such a method for determining the microbiological contamination of a surface. The method comprises joining the plate to the surface to be analyzed, such as for example by an adhesive, removing the plate from the surface, e.g. by cutting the adhesive, after a given period of time and incubating the plate with a culture medium followed by the inspection of the growth of microbes in the culture medium. The convex shape of the plate is suitable for receiving the adhesive for proper attachment to the surface in the environment to be analyzed.

Although the use of small model surfaces renders this method highly reliable, the curved structure of the plate for containing the adhesive for direct attachment of the plate to the surface to be analyzed has the disadvantage that the adhesive may leak out of the boundaries of plate (the model surface) and become spread on the surface to be analyzed. This may adversely affect the surface to be analyzed, especially in the event such surface is from food.

### SUMMARY OF THE INVENTION

The present invention provides a method for determining the microbiological contamination in an environment with which it is possible to avoid the above mentioned disadvantage and provides further advantages as it will be explained hereinbelow.

The invention further refers to a device for determining the microbiological contamination in an environment carrying out said environment.

As used herein, the term "environment" is intended to comprise all the items surrounded under substantially the same certain conditions, which items may include areas, portions or surfaces of items, goods, objects, devices, etc. placed therein.

The method of the invention comprises the steps of providing one or more supporting members in the environment to be analyzed, such as for example a foodstuff processing facility, restaurants, industries, hospitals, and the like. This determination is useful for, e.g., maintaining a hygienic state of the environment concerned.

Each supporting member is suitable for detachably receiving a corresponding sampling plate, so the supporting members may be provided into the environment already with the corresponding sampling plate fitted therein or with said corresponding sampling plate being provided at a later step, once the supporting member has been arranged in the environment. A subsequent step is performing for removing the sampling plate from the supporting member after a period of time.

Therefore, the step of removing the sampling plate from the supporting member is carried out such that the microbiological contamination in said environment can be determined by analyzing said sampling plate through any suitable microbiological analysis. Such period of time in said sampling plate removal step depends on the conditions of the environment and it will be enough to ensure contamination.

The sampling plate analysis, once detached from the supporting member, may be carried out by using, for example, a sterile Petri dish with culture medium (for example, PCA or TSA) that may be added such that, when incubated in an oven, the microbiological growth can be assessed.

In one embodiment of the method of the invention, an initial step may be further carried out consisting in fixing the supporting member to a surface in said environment through suitable means.

The invention further relates to a device for determining the microbiological contamination in an environment which may perform the method as disclosed.

The device of the invention comprises a supporting member that is adapted for being fixed to a surface in said environment. The device of the invention is designed for detachably receiving at least one sampling plate (also referred to as model surface or test surface). The sampling plate is sized to be fixed on said supporting member for determining the level of contamination thereof. More particularly, the sampling plates are shaped to be fixed on respective complementarily shaped recess formed in said supporting member.

The sampling plate may be a small plate or disc that may be, e.g. a sterile plate (made, for example, of ferritic steel or other suitable materials such as stainless steel, PVC, wood, plastic or tissue). It will be understood that other sizes and shapes for the sampling plate might be used. In one example, the sampling plate is a 1-10 cm (e.g. 2 cm) diameter disc.

The device may further include attaching means (e.g. a suitable adhesive or at least one magnet) for detachably fixing the sampling plate in the supporting member. In the embodiment in which the attaching means comprise a magnet, said magnet may be at least part of the supporting member. The supporting member of the device may be further provided with at least one recess for receiving said attaching means for the sampling plates.

Releasing means may be also provided for enabling or easing the removal of the sampling plate from the supporting member, more particularly from the respective recess thereof. Said releasing means may comprise at least one cavity formed in a portion of the supporting member where the sampling plate is seated. This cavity is adapted for allowing the sampling plate to be inclined when a peripheral portion thereof is pressed down.

In one embodiment of the device of the invention, it may further include attaching means for fixing the supporting member to said surface in the environment. Said attaching means may be, for example, a suitable adhesive. Other suitable known attaching means may alternatively be used.

The device disclosed according to the invention may be used to perform the above mentioned method. In this respect, a plurality of devices may be arranged for determining the microbiological contamination in an environment (and consequently all the objects, areas and/or surfaces being surrounded and under the conditions therein), with each device carrying one or several sampling plates and with each sampling plate being capable to detachably receive one or several sampling plates. Devices may be distributed within the environment to be analyzed according to the requirements.

Through the method and the device of the invention, a simple and effective way to determine the microbiological contamination of one or more areas in an environment is achieved. It has been shown that through the method and the device of the invention the microbiological contamination can be easily determined and monitored, particularly for assessing possible sources of contamination, the evolution of microbiological contamination, in foodstuffs, pharmaceuticals and any other products requiring an environmental control of bacteria.

### BRIEF DESCRIPTION OF THE DRAWINGS

A particular embodiment of method and the device for determining the microbiological contamination in an environment according to the present invention will be described in the following, only by way of non-limiting example, with reference to the appended drawings, in which:
Fig. is a top plan view of a first embodiment of a device for determining the microbiological contamination in an environment according to the invention;
Fig. 2 is a front sectional view of the first embodiment of the device in Fig. 1 taken along line AA in Fig. 1;
Fig. 3 is a top plan view of a second embodiment of a device for determining the microbiological contamination in an environment according to the invention;
Fig. 4 is a front sectional view of the second embodiment of the device in Fig. 2 taken along line BB in Fig. 3; and
Fig. 5 is a side sectional view of the second embodiment of the device in Fig. 2 taken along line CC in Fig. 3.

### DETAILED DESCRIPTION OF PARTICULAR EMBODIMENTS

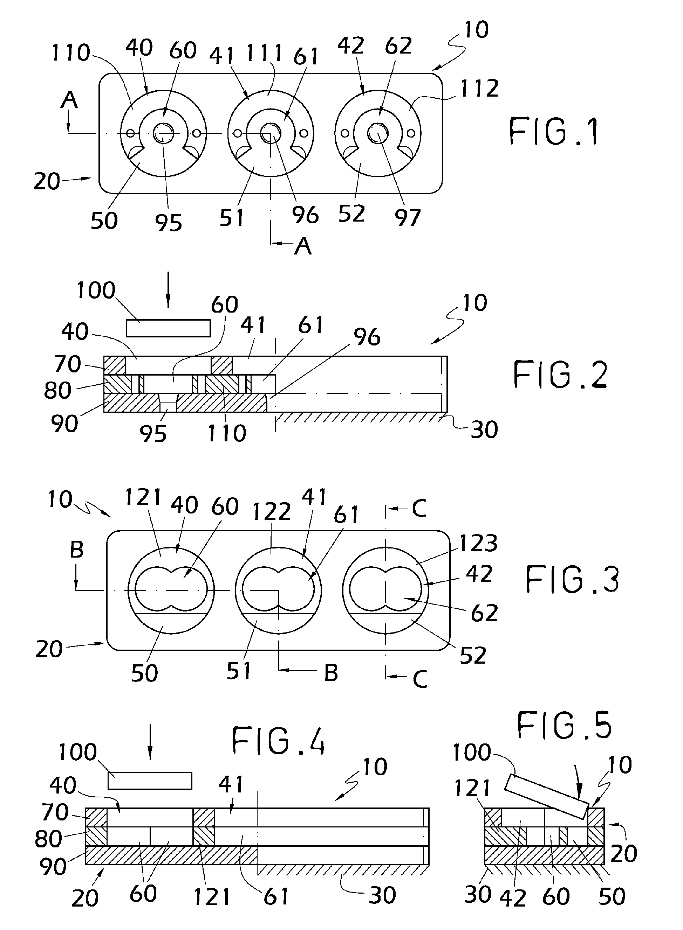

According to the figures 1 and 2 of the drawings, a first embodiment of a device for determining the microbiological contamination in an environment according to the invention is shown designated generally through reference numeral 10.

The device 10 comprises a supporting member 20 made, for example, of a suitable stainless steel. The supporting member 20 is adapted for being fixed to a surface 30 as diagrammatically shown in Fig. 2. The surface 30 may be part of an object which microbiological contamination is to be assessed. The supporting member 20 may be made of a single piece or it could be made, as shown in the figures, of several metal layers 70, 80, 90 as shown in Fig. 2.

The supporting member 20 of the device 10 is provided with an upper series of recesses 40, 41, 42 formed in an upper metal layer 70 of the supporting member 20. Although in the embodiment shown, three recesses are provided, it will be apparent that the device may be provided with a supporting member 20 having a different number of recesses.

Recesses 40, 41, 42 are sized and adapted for detachably receiving corresponding sampling plates 100 which are to be used as model or test surfaces. In the embodiment shown in the figures, both the recesses 40, 41, 42 (and consequently the sampling plates 100) are circular in shape. Other suitable shapes for the recesses 40, 41, 42 and the sampling plates 100, as well as other number thereof, may of course be provided. In the embodiment shown, the sampling plates 100 are in the form of small sterile discs made of steel.

In the embodiment shown in the figures, the upper series of recesses 40, 41, 42 are each provided with an opening defining a cavity 50, 51, 52. Each of said cavities 50, 51, 52 brings the upper series of recesses 40, 41, 42 in communication with a lower series of recesses 60, 61, 62 formed in an intermediate metal layer 80 of the supporting member 20, that is, arranged beneath said upper series of recesses 40, 41, 42. The cavities 50, 51, 52 in the upper series of recesses 40, 41, 42 serve the purpose of acting as a releasing means for enabling or easing the removal of the sampling plate 100 from the supporting member 20. By pressing a peripheral portion of the sampling plate 100 down towards the corresponding cavity 50, 51, 52 in respective recess 40, 41 42 (see Fig. 5), the sampling plate 100 is forced to be inclined allowing the sampling plate 100 to be detached from the respective recess 40, 41, 42 into which it is fitted.

The lower series of recesses 60, 61, 62 are sized to receive attaching means which may be, for example, an adhesive or a magnet. The attaching means serves the purpose of detachably fixing the sampling plate 100 in the supporting member 20.

The supporting member 20 comprises a third, lower metal layer 90 on which the intermediate and the upper layers 80, 70 are arranged, respectively. Said inner layer 90 of the supporting member 20 is provided with center holes 95, 96, 97 formed according to the respective recesses 40, 41 42 and 60, 61, 62.

A second embodiment of the invention is shown figures 3-5. This second embodiment of the invention differs from the first embodiment in Figs. 1-2 in the construction of the recesses 40, 41 42 and 60, 61, 62: while in the first embodiment shown in figs. 1 and 2 of the drawings, the recess 40, 41, 42 are formed by providing an intermediate C-shaped member 111, 112, 113 arranged in correspondence with the intermediate layer 80, in the second embodiment shown in figs. 3, 4 and 5 of the drawings, an intermediate member 121, 122, 123 having two contiguous holes, defining the recesses 60, 61, 62, is arranged in correspondence with the intermediate layer 80.

With one or more devices 10 as disclosed, a method for determining the microbiological contamination in an environment can be carried out. The method consists in providing one or more of said devices 10 into an environment to be analyzed by attaching them to a surface 30 through the supporting member 20. The supporting member 20 may be already provided with the corresponding sampling plates 100 or they may be fitted once the supporting member 20 is attached to the surface 30 in the environment. After a suitable period of time, the sampling plates 100 are detached from the respective supporting member 20 of the device 10 such that it can be analyzed for microbiological contamination through any suitable microbiological analysis. The sampling plate analysis may be carried out by using, for example, a sterile Petri dish with culture medium (for example, PCA or TSA) that may be added so that, when incubated in an oven, the microbial growth can be assessed.

The period of time in said sampling plate removal step depends on the conditions of the environment which will be enough to ensure contamination.

## Claims

1. Method for determining the microbiological contamination in an environment that comprises the step of providing at least one sampling plate (100) into said environment, **characterized in that** if further comprises the step of providing at least one supporting member (20) in said environment suitable for detachably receiving said sampling plate (100) and a subsequent step of removing the sampling plate (100) from the supporting member (20) after a period of time such that the microbiological contamination in said environment can be determined by analyzing said sampling plate (100).

2. Method as claimed in claim 1, wherein it further includes an initial step of fixing the supporting member (20) to a surface (30) in said environment.

3. Device (10) for determining the microbiological contamination in an environment **characterized in that** it comprises a supporting member (20) that is adapted for being fixed to a surface (30) in said environment and adapted for detachably receiving at least one sampling plate (100).

4. Device (10) as claimed in claim 3, wherein if further comprises attaching means for detachably fixing the sampling plate in the supporting member.

5. Device (10) as claimed in claim 4, wherein said attaching means is an adhesive.

6. Device (10) as claimed in claim 4, wherein said attaching means comprises a magnet.

7. Device (10) as claimed in claim 6, wherein said magnet is at least part of the supporting member (20).

8. Device (10) as claimed in any of the claims 3 to 7, wherein the supporting member is provided with at least one recess (60, 61, 62) for receiving the attaching means.

9. Device (10) as claimed in any of the claims 3 to 8, wherein it further includes releasing means (50, 51, 52) for removing of the sampling plate (100) from the supporting member (20).

10. Device as claimed in claim 9, wherein said releasing means (50, 51, 52) comprise at least one cavity formed in a portion of the supporting member (20) where the sampling plate is seated adapted for allowing the sampling plate (100) to be inclined when pressing it down.

11. Device as claimed in any of the claims 3 to 10, wherein it further includes attaching means for fixing the supporting member (20) to said surface (30) in the environment.

12. Device as claimed in any of the claims 3 to 11, wherein said sampling plate (100) is a sterile plate.
